(19) Europäisches Patentamt
European Patent Office
Office européen des brevets

(11) Veröffentlichungsnummer: **0 230 230**
**A2**

# (12) EUROPÄISCHE PATENTANMELDUNG

(21) Anmeldenummer: **87100170.7**

(51) Int. Cl.4: **C07C 131/00**

(22) Anmeldetag: **09.01.87**

(30) Priorität: **21.01.86 DE 3601564**

(43) Veröffentlichungstag der Anmeldung: **29.07.87 Patentblatt 87/31**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Elbe, Hans-Ludwig, Dr.**
**Dasnöckel 59**
**D-5600 Wuppertal 11(DE)**
Erfinder: **Lantzsch, Reinhard, Dr.**
**Am Buschhäuschen 51**
**D-5600 Wuppertal 1(DE)**

(54) **Verfahren zur Herstellung von Hydroxybenzaldoxim-O-ethern.**

(57) Nach einem neuen Verfahren werden bekannte Hydroxybenzaldoxim-O-ether der Formel

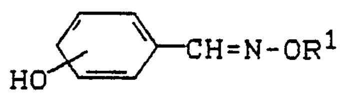

(I)

in welcher
R' für Alkyl steht,
dadurch erhalten, daß man Hydroxybenzaldoxime der Formel

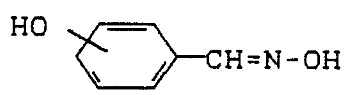

(II)

mit Dialkylsulfaten der Formel
$R^1-O-SO_2-O-R^1$ (III)
in welcher
R' die oben angegebene Bedeutung hat,
oder mit Alkylhalogeniden der Formel
R'X (IV)
in welcher
R' die oben angegebene Bedeutung hat und
X für Halogen steht,
in Gegenwart einer starken Base und in Gegenwart eines Verdünnungsmittels bei Temperaturen zwischen 0° C und 60 °C umsetzt.

EP 0 230 230 A2

## Verfahren zur Herstellung von Hydroxybenzaldoxim-O-ethern

Die Erfindung betrifft ein neues Verfahren zu Herstellung von bekannten Hydroxybenzaldoxim-O-ethern, die als Zwischenprodukte zur Synthese von Verbindungen mit fungizider, insektizider und antimykotischer Wirksamkeit verwendet werden können.

Es ist bereits bekannt geworden, daß man bestimmte Hydroxybenzaldoxim-O-ether herstellen kann, indem man Hydroxybenzaldehyde mit den entsprechenden Hydroxylamin-Derivaten umsetzt (vgl. EP-OS 0 076 370 und EP-OS 0 115 828). Die betreffende Umsetzung läßt sich durch das folgende Reaktionsschema veranschaulichen:

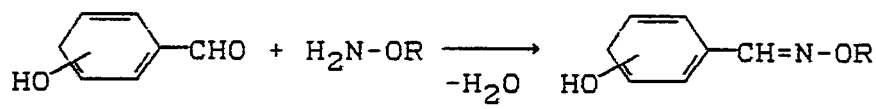


R = Alkyl, Alkenyl, Alkinyl.

Nachteilig an diesem Verfahren sind jedoch der hohe Preis und die schlechte Verfügbarkeit der als Reaktionskomponenten benötigten substituierten Hydroxylamine. Der Einsatz dieser Stoffe für eine Herstellung von Hydroxybenzaldoxim-O-ethern in technischem Maßstab ist daher problematisch.

Weiterhin ist bekannt, daß O-substituierte Aldoxime in einem technischen Prozeß erhalten werden können, wenn man Oxime mit einem Organochlorid und einem Alkalimetall-Hydroxid in einem niedermolekularen Alkohol umsetzt (vgl. EP-OS 0 121 701). Als Substituenten im Phenylteil kommen hierbei jedoch nur solche Reste in Frage, die sich unter den Reaktionsbedingungen inert verhalten. Die Umsetzung von Hydroxybenzaldoximen ist daher unter diesen Bedingungen nicht möglich.

Es wurde nun gefunden, daß man bekannte Hydroxybenzaldoxim-O-ether der Formel

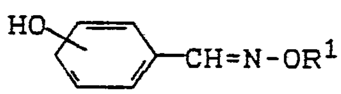
(I)

in welcher
$R^1$ für Alkyl steht,
erhält, wenn man Hydroxybenzaldoxime der Formel

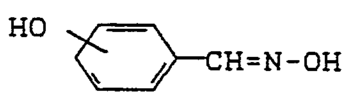
(II)

mit Dialkylsulfaten der Formel
$R^1-O-SO_2-O-R^1$ (III)
in welcher
$R^1$ die oben angegebene Bedeutung hat,
oder mit Alkylhalogeniden der Formel
$R^1X$ (IV)
in welcher
$R^1$ die oben angegebene Bedeutung hat und
X für Halogen steht,
in Gegenwart einer starken Base und in Gegenwart eines Verdünnungsmittels bei Temperaturen zwischen 0° C und 60° C umsetzt.

Es ist als äußerst überraschend zu bezeichnen, daß die erfindungsgemäße Umsetzung unter den angegebenen Verfahrensbedingungen glatt und in guter Ausbeute abläuft. Es war nämlich zu erwarten, daß das Alkylierungsmittel in sehr starkem Maß an der phenolischen OH-Gruppe angreift, da diese in Gegenwart einer starken Base als nukleophiles Phenol-Anion vorliegt.

Das erfindungsgemäße Verfahren zeichnet sich durch eine Reihe von Vorteilen aus. So ermöglicht es die Herstellung von Hydroxybenzaldoxim-O-ethern der Formel (I) in hohen Ausbeuten, wobei gut zugängliche Verbindungen als Ausgangsprodukte eingesetzt werden. Ferner ist die Umsetzung einfach durchführbar, und die Isolierung der Hydroxybenzaldoxim-O-ether der Formel (I) bereitet keinerlei Schwierigkeiten. Das erfindungsgemäße Verfahren ist somit besonders geeignet, um Hydroxybenzaldoxim-O-ether auch in technischem Maßstab herzustellen.

Verwendet man beispielsweise 4-Hydroxybenzaldoxim als Ausgangsstoff, Diethylsulfat als Alkylierungsmittel, Natriumhydroxid als starke Base und Wasser als Verdünnungsmittel, so kann der Verlauf des erfindungsgemäßen Verfahrens durch das folgende Formelschema veranschaulicht werden:

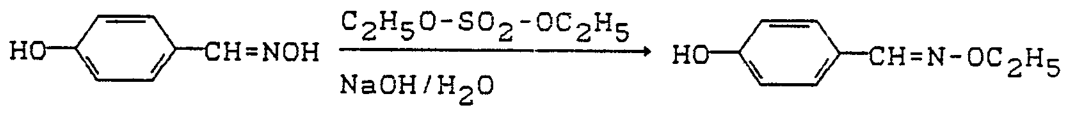


Verwendet man 4-Hydroxybenzaldoxim-hydrat als Ausgangsmaterial, Methylchlorid als Alkylierungsmittel, Natriumhydroxid als starke Base und Wasser als Verdünnungsmittel, so kann der Verlauf des erfindungsgemäßen Verfahrens durch das folgende Formelschema veranschaulicht werden:

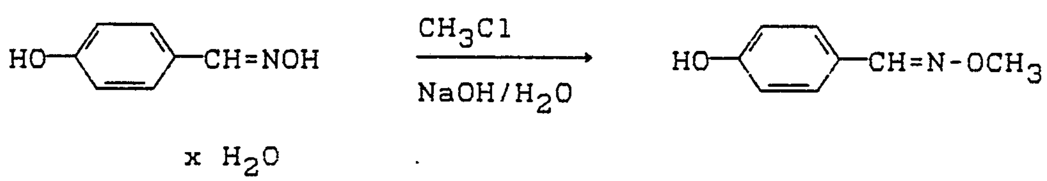


Die bei dem erfindungsgemäßen Verfahren als Ausgangsstoffe zu verwendenden Hydroxybenzaldoxime sich durch die Formel (II) allgemein definiert. Besonders bevorzugt ist das 4-Hydroxybenzaldoxim bzw. dessen Hydrat.

Die Hydroxybenzaldoxime der Formel (II) sind allgemein bekannte Verbindungen der organischen Chemie (vgl. z.B. EP-OS 0 012 158, CA 96:68526p oder CA 85:21226y). Sie können in besonders vorteilhafter Weise durch Umsetzung von Hydroxybenzaldehyden mit Hydroxylaminsulfat-Lösung erhalten werden .

Die für das erfindungsgemäße Verfahren außerdem als Ausgangsstoffe zu verwendenden Dialkylsulfate sind durch die Formel (III) allgemein definiert. In dieser Formel steht R' vorzugsweise für geradkettiges oder verzweigtes Alkyl mit 1 bis 10 Kohlenstoffatomen.

Besonders bevorzugte Ausgangsstoffe sind diejenigen Dialkylsulfate der Formel (III), in denen R' für geradkettiges oder verzweigtes Alkyl mit 1 bis 4 Kohlenstoffatomen steht.

Die bei dem erfindungsgemäßen Verfahren außerdem als Ausgangsstoffe zu verwendenden Alkylhalogenide sind durch die Formel (IV) allgemein definiert. Auch in dieser Formel steht R' vorzugsweise für geradkettiges oder verzweigtes Alkyl mit 1 bis 10 Kohlenstoffatomen, insbesondere mit 1 bis 4 Kohlenstoffatomen. X steht vorzugsweise für Chlor, Brom oder Iod.

Die Dialkylsulfate der Formel (III) und die Alkylhalogenide der Formel (IV) sind allgemein bekannte Verbindungen der organischen Chemie.

Das erfindungsgemäße Verfahren wird in Gegenwart eines Verdünnungsmittels durchgeführt. Hierfür kommen vorzugsweise polare, protische Lösungsmittel infrage, wie Alkohole, z.B. Methanol, Ethanol, n-und i-Propanol und n-und i-Butanol; ferner Wasser sowie Mischungen von Wasser mit den genannten organischen Lösungsmitteln.

Als starke Basen Kommen für das erfindungsgemäße Verfahren vorzugsweise Natriumhydroxid und Kaliumhydroxid infrage.

Die Temperaturen können bei der Durchführung des erfindungsgemäßen Verfahrens in einem größeren Bereich variiert werden. Im allgemeinen arbeitet man bei Temperaturen zwischen 0° C und 60° C, vorzugsweise zwischen 10° C und 50° C.

Wenn als Alkylierungsmittel gasförmige Alkylhalogenide der Formel (IV) eingesetzt werden, so arbeitet man in Druckgefäßen unter einem Druck zwischen 1 und 15 bar.

Bei der Durchführung des erfindungsgemäßen Verfahrens setzt man auf 1 Mol Hydroxybenzaldoxim der Formel (II) 1 bis 2 Mol, vorzugsweise 1 bis 1,5 Mol Dialkylsulfat der Formel (III) und 1 bis 4 Mol, vorzugsweise 1,5 bis 3 Mol Base ein.
Die Reaktionszeit beträgt im allgemeinen 5 bis 15 Stunden, vorzugsweise 5 bis 10 Stunden.
Die Isolierung der Hydroxybenzaldoxim-O-ether der Formel (I) erfolgt in üblicher Art und Weise, wie beispielsweise durch fraktionierte Destillation.

Die nach dem erfindungsgemäßen Verfahren herstellbaren Hydroxybenzaldoxim-O-ether der Formel (I) sind allgemein bekannte Ausgangsstoffe zur Synthese von biologisch aktiven Verbindungen, wie z.B. zur Synthese von Oximethern, welche gute insektizide Eigenschaften besitzen (vgl. EP-OS 0 115 828); von Azolyl-phenoxy-Derivaten, welche hervorragende fungizide Eigenschaften aufweisen (vgl. EP-OS 0 076 370); von 1-Hydroxyethyl-triazolyl-Derivaten, welche gute fungizide und antimykotische Eigenschaften aufweisen (vgl. EP-OS 0 110 048 und DE-OS 3 314 548); sowie von Hydroxyalkyl-azol-Derivaten, welche gute antimykotische Wirksamkeit besitzen (vgl. DE-OS 3 427 844).

So läßt sich beispielsweise das 3,3-Dimethyl-1-(4-methoximinomethyl-phenoxy)-1-(1,2,4-triazol-1-yl)-butan-2-on der Formel

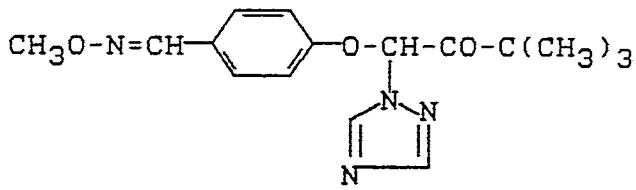

herstellen, indem man 3,3-Dimethyl-1-(1,2,4-triazol-1-yl)-butan-2-on zunächst mit Brom zum 1-Brom-(1,2,4-triazol-1-yl)-3,3-dimethyl-butan-2-on umsetzt und dieses anschliessend mit 4-Hydroxybenzaldehyd-O-methyl-oximether in Gegenwart einer Base umsetzt. Diese Synthese läßt sich formelmäßig wie folgt veranschaulichen:

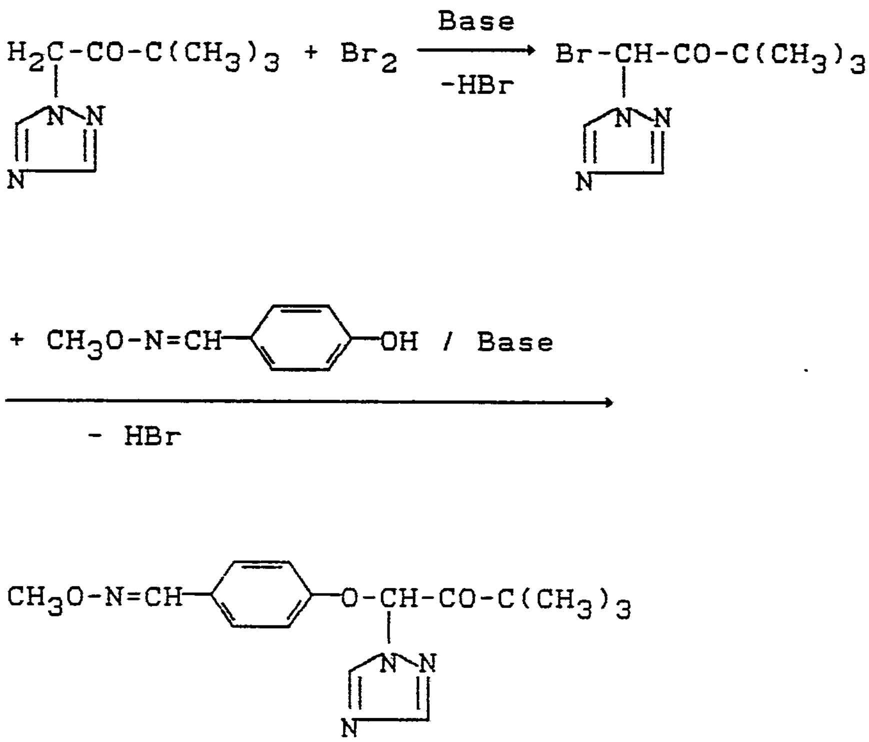

Das erfindungsgemäße Verfahren wird durch die nachfolgenden Beispiele veranschaulicht.

Beispiel 1

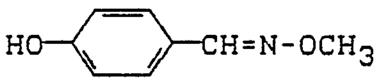

(I-1)

318,4 g (1.889 Mol) 4-Hydroxybenzaldoxim-hydrat (92 %-ig) und 197,2 g (4.93 Mol) Natriumhydroxid werden in 2 l Wasser beim Raumtemperatur vorgelegt. Man läßt 15 Minuten nachrühren und versetzt anschließend mit 285,5 g (2;266 Mol) Dimethylsulfat. Nach erfolgter Zugabe läßt man 8 Stunden bei Raumtemperatur nachrühren. Das Reaktionsgemisch wird mit verdünnter wäßriger Schwefelsäure neutralisiert und mit Methylenchlorid extrahiert. Die organische Phase wird getrocknet und im Vakuum eingeengt. Durch gaschromatographische Bestimmung wird ermittelt, daß das so anfallende Rohprodukt folgende Stoffe in den angegegebenen prozentualen Mengen enthält:

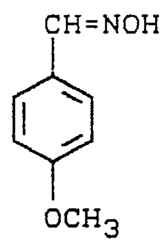

:

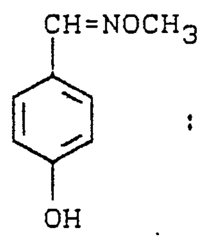

:

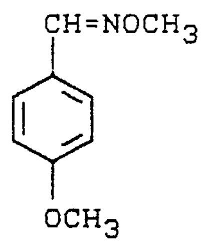

5,1 % : 73,7 % : 13,4 %

Dieses Rohprodukt wird fraktioniert destilliert. Man erhält 179,4 g (62,9 % der Theorie) 4-Methoximinomethylphenol vom Siedepunkt 143 °C/0,13 mbar.

Beispiel 2

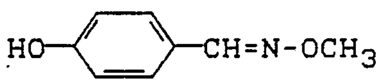

(I-1)

Eine Lösung von 137 g (0,89 Mol) 4-Hydroxybenzaldoxim-Hydrat und 104 g (2,6 Mol) Natriumhydroxid in 715 ml Wasser wird bei Raumtemperatur in einem 1,3 l VA-Autoklaven gegeben. Man drückt 56 g (= 61,5 ml; 1,1 Mol) Methylchlorid auf und erhitzt auf 50° C, wobei sich ein Druck von 10 bar einstellt. Nach fünfstündigem Rühren bei 50° C ist der Druck auf 5 bar gefallen. Man entspannt auf Normaldruck und stellt den pH-Wert des Reaktionsgemisches durch Zugabe von verdünnter, wäßriger Schwefelsäure auf 6 ein. Anschließend filtriert man das Reaktionsgemisch, extrahiert das Filtrat mit Methylenchlorid, trocknet die vereinigten organischen Phasen und engt unter vermindertem Druck ein. Es verbleiben 103,7 g eines Rohproduktes, das einer fraktionierten Destillation unterworfen wird. Man erhält auf diese Weise 84,7 g eines Produktes, das zu 93 % aus 4-Methoximinomethyl-phenol besteht. Die Ausbeute errechnet sich danach zu 58,6 % der Theorie. Siedepunkt: 124 bis 128° C/0,07 mbar.

Herstellung der Verbindung der Formel:

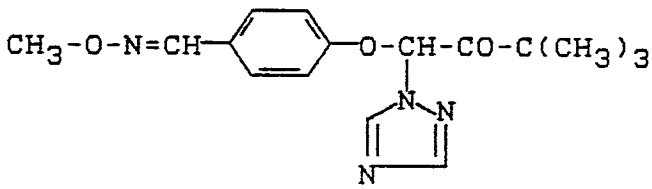

In eine Lösung von 217 g (1,3 Mol) 3,3-Dimethyl-1-(1,2,4-triazol-1-yl)-2-butanon in 700 ml Eisessig trägt man 110 g (1,34 Mol) Natriumacetat ein, wobei die Temperatur auf ca. 28° C ansteigt. Man läßt 30 Minuten nachrühren und versetzt tropfenweise unter leichter Kühlung bei 30 bis 33° C mit 208 g (1,3 Mol) Brom. Das Reaktionsgemisch wird 2,5 Stunden bei Raumtemperatur nachgerührt und in 1200 ml Wasser gegeben. Man extrahiert mit Methylenchlorid, wäscht mit Wasser und wäßriger Bicarbonatlösung, trocknet über Natriumsulfat und engt ein.

Das so erhaltene rohe 1-Brom-3,3-Dimethyl-1-(1,2,4-triazol-1-yl)-2-butanon wird in 100 ml Acetonitril gelöst und zu einer Aufschlämmung von 151 g (1 Mol) 4-Methoximinomethylphenol und 150 g (1,09 Mol) Kaliumcarbonat in 800 ml Acetonitril gegeben, wobei die Temperatur auf etwa 40° C ansteigt. Man läßt das Reaktionsgemisch 3 Stunden bei 60 bis 65° C nachrühren, kühlt anschließend ab und gibt auf Wasser. Man extrahiert mit Toluol, wäscht mit Wasser, trocknet und engt ein. Der Rückstand wird in Ligroin verrieben und auf Ton getrocknet. Man erhält 241 g (76 % der Theorie) 3,3-Dimethyl-1-(4-methoximinomethyl-phenoxy)-1-(1,2,4-triazol-1-yl)-butan-2-on vom Schmelpunkt 83-87° C.

**Ansprüche**

1. Verfahren zur Herstellung von Hydroxybenzaldoxim-O-ethern der Formel

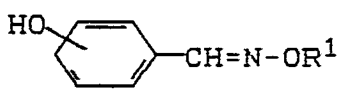 (I)

in welcher
$R^1$ für Alkyl steht,
dadurch gekennzeichnet, daß man Hydroxybenzaldoxime der Formel

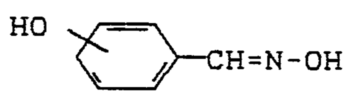 (II)

mit Dialkylsulfaten der Formel
$R^1$ -O -$SO_2$ -O -$R^1$ (III)
in welcher
$R^1$ die oben angegebene Bedeutung hat,
oder mit Alkylhalogeniden der Formel
$R^1X$ (IV)
in welcher
$R^1$ die oben angegebene Bedeutung hat und
X für Halogen steht,
in Gegenwart einer starken Base und in Gegenwart eines Verdünnungsmittels bei Temperaturen zwischen 0° C und 60° C umsetzt.

2. Verfahren gemäß Anspruch 1, dadurch gekennzeichnet, daß man als Ausgangsstoff der Formel (II) 4-Hydroxybenzaldoxim einsetzt.

3. Verfahren gemäß Anspruch 1, dadurch gekennzeichnet, daß man Dialkylsulfate der Formel (III) oder Alkylhalogenide der Formel (IV) einsetzt, in denen $R^1$ für geradkettiges oder verzweigtes Alkyl mit 1 bis 10 Kohlenstoffatomen steht.

4. Verfahren gemäß Anspruch 1, dadurch gekennzeichnet, daß man Dialkylsulfate der Formel (III) oder Alkylhalogenide der Formel (IV) einsetzt, in denen $R^1$ für geradkettiges oder verzweigtes Alkyl mit 1 bis 4 Kohlenstoffatomen steht.

5. Verfahren gemäß Anspruch 1, dadurch gekennzeichnet, daß man Dialkylsulfate der Formel (III) oder Alkylhalogenide der Formel (IV) einsetzt, in denen $R^1$ für Methyl oder Ethyl steht.

6. Verfahren gemäß Anspruch 1, dadurch gekennzeichnet, daß man Alkylhalogenide der Formel (IV) einsetzt, in denen X für Chlor, Brom oder Iod steht.

7. Verfahren gemäß Anspruch 1, dadurch gekennzeichnet, daß man Natriumhydroxid oder Kaliumhydroxid als starke Basen einsetzt.

8. Verfahren gemäß Anspruch 1, dadurch gekennzeichnet, daß man Alkohole, Wasser oder deren Mischungen als Verdünnungsmittel einsetzt.

9. Verfahren gemäß Anspruch 1, dadurch gekennzeichnet, daß man bei Temperaturen zwischen 10° C und 50° C arbeitet.

10. Verfahren gemäß Anspruch 1, dadurch gekennzeichnet, daß man auf 1 Mol an Hydroxybenzaldoxim der Formel (II) 1 bis 2 Mol Dialkylsulfat der Formel (III) oder Alkylhalogenid der Formel (IV) und 1 bis 4 Mol an Base einsetzt.